# FASCICULE DE BREVET EUROPEEN

(11) **EP 1 426 040 B1**
(45) Date de publication et mention de la délivrance du brevet: **28.09.2011**
(21) Numéro de dépôt: 03293025.7
(22) Date de dépôt: 04.12.2003
(51) Int. Cl.: A61K 8/73, A61K 8/41, A61Q 5/10

(54) **Composition de teinture d'oxydation pour fibres kératiniques comprenant un colorant d'oxydation, un polymère associatif, un composé cellulosique non ionique ne comportant pas de chaine grasse en C8 - C30 et un polymère cationique de densité de charge supérieure à 1meq/g et ne comportant pas de chaine grasse en C8 - C30**
Oxidative Zusammensetzung enthaltend ein Oxidationsmittel, ein assoziatives Polymer, eine nichtionische cellulosehaltige Verbindung ohne C8-30 Fettkette und ein kationisches Polymer mit einer Ladungsdichte grösser als 1 meq/g ohne C8-30 Fettkette
Oxidising composition for keratin fibres comprising an oxidising agent, an associative polymer, a nonionic cellulosic compound having no C8-30 fatty chain and a cationic polymer having a charge density greater than 1 meq/g and having no C8-30 fatty chain

(30) Priorité: 06.12.2002 FR 0215469
(43) Date de publication de la demande: 09.06.2004
(60) Demande divisionnaire: 10183819.1
(73) Titulaire: L'Oréal, 75008 Paris (FR)
(72) Inventeur: Simonet, Frédéric, 77131 Touquin (FR); Nicolas-Morgantini, Luc, 60810 Rully (FR); Cottard, Francois, 92400 Courbevoie (FR); Rondeau, Christine, 78500 Sartrouville (FR)
(74) Mandataire: Casalonga, Axel

(56) Documents cités:
- FR-A- 2 802 089
- FR-A- 2 816 207
- FR-A- 2 817 466
- FR-A- 2 817 467
- FR-A- 2 820 032
- US-A- 6 156 076
- US-A1- 2001 023 515
- US-A1- 2002 046 431

## Description

La présente invention est relative à une composition de teinture d'oxydation des fibres kératiniques, en particulier des fibres kératiniques humaines et plus particulièrement des cheveux, comprenant au moins un colorant d'oxydation, au moins un polymère associatif, au moins un composé cellulosique non ionique ne comportant pas de chaîne grasse en C₈ - C₃₀ et au moins un polymère cationique de densité de charge supérieure à 1 meq/g et ne comportant pas de chaîne grasse en C₈ - C₃₀.

Il est connu de teindre les fibres kératiniques, et en particulier les cheveux humains, avec des compositions de teinture contenant des précurseurs de colorants d'oxydation, généralement appelés « bases d'oxydation », en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des « bases d'oxydation » sur elles-mêmes, soit d'une condensation oxydative des « bases d'oxydation » sur des composés modificateurs de coloration, ou « coupleurs », qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les « bases d'oxydation » et d'autre part par les « coupleurs », permet l'obtention d'une palette très riche en coloris.

Les compositions de teinture d'oxydation comprennent en outre généralement des polymères cationiques afin d'améliorer les propriétés cosmétiques.

Cependant, la demanderesse a constaté que les compositions tinctoriales comprenant ces polymères cationiques étaient peu stables, présentaient de propriétés cosmétiques peu satisfaisantes, et peu rémanentes aux shampooings.

Or, après d'importantes recherches menées sur la question, la demanderesse vient maintenant de découvrir que des compositions de teinture d'oxydation comprenant un colorant d'oxydation, un polymère associatif cationique particulier un composé cellulosique non ionique ne comportant pas de chaîne grasse en C₈ - C₃₀ et un polymère cationique de densité de charge supérieure à 1 meq/g et ne comportant pas de chaîne grasse en C₈ - C₃₀ présentent une bonne stabilité physico-chimique, ainsi que de bonnes propriétés cosmétiques, en particulier la douceur, le lissage, la souplesse, la légèreté.

Elle a également constaté que lesdites compositions conféraient des propriétés cosmétiques rémanentes aux shampooings. Elle a ainsi observé une très bonne rémanence de la couleur aux shampooings.

Elle a également constaté que lesdites compositions permettaient d'obtenir une sélectivité faible, c'est-à-dire des écarts de coloration faibles tout au long d'une même fibre kératinique, qui peut être différemment sensibilisée (i.e. abîmée) entre sa pointe et sa racine.

Elle a enfin constaté que lesdites compositions présentaient une agressivité moindre vis-à-vis de l'environnement, du fait de l'emploi de cellulose non ionique.

La présente invention a ainsi pour objet une composition pour la teinture d'oxydation des fibres kératiniques, en particulier des fibres kératiniques humaines, telles que les cheveux, caractérisée en ce qu'elle comprend, dans un milieu approprié pour la teinture,
a) au moins un colorant d'oxydation,
b) au moins un polymère associatif, cationique choisi parmi les celluloses quaternisées modifiées par des groupements comportant au moins une chaîne grasse et les hydroxyéthylcelluloses comportant au moins une chaîne grasse
c) au moins un composé cellulosique non ionique ne comportant pas de chaîne grasse en C₈- C₃₀, et
d) au moins un polymère cationique de densité de charge supérieure à 1 meq/g et ne comportant pas de chaîne grasse en C₈ - C₃₀.

Un autre objet de l'invention porte sur une composition prête à l'emploi pour la teinture des fibres kératiniques qui comprend au moins un colorant d'oxydation, au moins un polymère associatif, au moins un composé cellulosique non ionique ne comportant pas de chaîne grasse en C₈ - C₃₀, au moins un polymère cationique de densité de charge supérieure à 1 meq/g et ne comportant pas de chaîne grasse en C₈ - C₃₀ et un agent oxydant.

Par « composition prête à l'emploi », on entend, au sens de l'invention, la composition destinée à être appliquée telle quelle sur les fibres kératiniques, c'est-à-dire qu'elle peut être stockée telle quelle avant utilisation ou résulter du mélange extemporané de deux ou plusieurs compositions.

L'invention vise également un procédé de teinture d'oxydation des fibres kératiniques, et en particulier des fibres kératiniques humaines telles que les cheveux, consistant à appliquer sur les fibres une composition (A) contenant, dans un milieu approprié pour la teinture, au moins un colorant d'oxydation, au moins un polymère associatif particulier au moins un composé cellulosique non ionique ne comportant pas de chaîne grasse en C₈ - C₃₀ et au moins un polymère cationique de densité de charge supérieure à 1 meq/g et ne comportant pas de chaîne grasse en C₈ - C₃₀, la couleur étant révélée à pH alcalin, neutre ou acide, à l'aide d'une composition (B) contenant au moins un agent oxydant, qui est mélangée juste au moment de l'emploi à la composition (A) ou qui est appliquée sur les fibres séquentiellement avant ou après la composition (A), sans rinçage intermédiaire.

L'invention a également pour objet des dispositifs de teinture à plusieurs compartiments ou « kits » à plusieurs compartiments pour la teinture d'oxydation des fibres kératiniques, en particulier des fibres kératiniques humaines telles que les cheveux. Un dispositif selon l'invention peut comporter un premier compartiment contenant au moins un colorant d'oxydation, au moins un polymère associatif particulier au moins un composé cellulosique non ionique ne comportant pas de chaîne grasse en C₈ - C₃₀ et au moins un polymère cationique de densité de charge supérieure à 1 meq/g et ne comportant pas de chaîne grasse en C₈ - C₃₀, et un deuxième compartiment contenant un agent oxydant.

D'autres caractéristiques, aspects, objets et avantages de l'invention apparaîtront encore plus clairement à la lecture de la description et des exemples qui suivent.

Par composé cellulosique, on entend selon l'invention tout composé polysaccharidique possédant dans sa structure des enchaînements de résidus glucose unis par des liaisons β-1,4.

Les composés cellulosiques non ioniques ne comportant pas de chaîne grasse en C₈ - C₃₀ de l'invention sont exclusivement choisis parmi les celluloses non substituées y compris sous une forme microcristalline et les éthers de cellulose.

Parmi les éthers de cellulose non ioniques ne comportant pas de chaîne grasse en C₈ - C₃₀, on peut citer les alkyl(C₁-C₄)celluloses, telles que les méthylcelluloses et les éthylcelluloses ; les hydroxyalkyl(C₁-C₄)celluloses, telles que les hydroxyméthylcelluloses ; les hydroxyéthylcelluloses et les hydroxypropylcelluloses ; les celluloses mixtes hydroxyalkyl(C₁-C₄)-alkyl(C₁-C₄)celluloses, telles que les hydroxypropyl-méthylcelluloses, les hydroxyéthyl-méthylcelluloses, les hydroxyéthyl-éthylcelluloses, et les hydroxbutyl-méthylcelluloses.

Le composé cellulosique non ionique ne comportant pas de chaîne grasse en C₈ - C₃₀ est présent dans la composition dans des proportions en poids de préférence comprises entre 0,1 et 10%, et encore de préférence entre 1 et 5% du poids total de la composition.

La densité de charge du polymère cationique peut être déterminée par la méthode KJELDAHL.

Au sens de la présente invention, l'expression "polymère cationique" désigne tout polymère contenant des groupements cationiques et/ou des groupements ionisables en groupements cationiques.

Les polymères cationiques de densité de charge supérieure à 1 meq/g et ne comportant pas de chaîne grasse en C₈-C₃₀ peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques de densité de charge supérieure à 1 meq/g et ne comportant pas de chaîne grasse en C₈ - C₃₀ préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques de densité de charge supérieure à 1 meq/g et ne comportant pas de chaîne grasse en C₈ - C₃₀ utilisés ont généralement une masse moléculaire moyenne en nombre comprise entre 500 et 5.10⁶ environ, et de préférence comprise entre 10³ et 3.10⁶ environ.

Parmi les polymères cationiques de densité de charge supérieure à 1 meq/g et ne comportant pas de chaîne grasse en C₈ - C₃₀, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer:
(1) Les homopolymères ou copolymères dérivés d'esters ou d'amides acryliques ou méthacryliques et comportant au moins un des motifs de formules (I), (II), (III) ou (IV) suivantes: 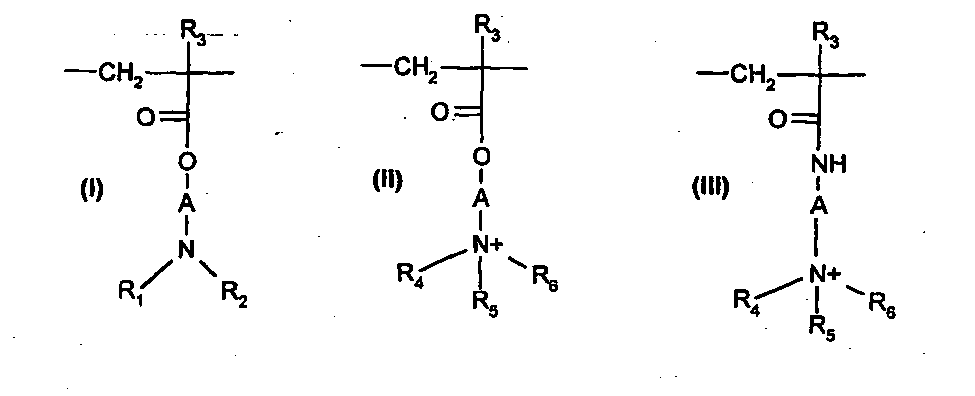 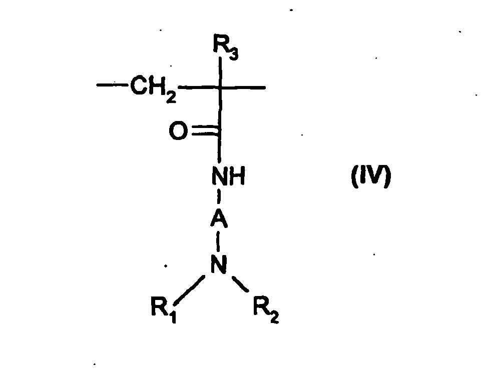 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 6 atomes de carbone;
   R₁ et R_{2,} identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques. Ainsi, parmi ces polymères de la famille (1), on peut citer :
- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
- les copolymères d'acrylamide et de chlorure de méthacryloyloxyéthyl-triméthylammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyl-triméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylamino-propyle quaternisés tel que le produit vendu sous la dénomination "GAFQUAT HS 100" par la société ISP.

(2) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(3) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(4) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(5) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 6 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(6) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 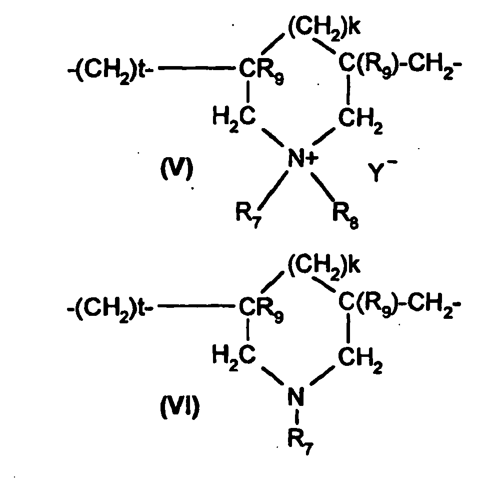 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(7) Le polymère de diammonium quaternaire contenant des motifs récurrents répondant à la formule : 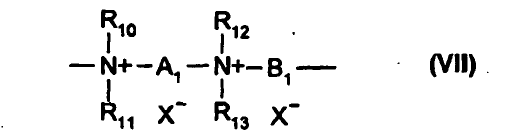 formule (VII) dans laquelle :
   R₁₀, R₁₁, R₁₂ et R₁₃, identiques ou différents, représentent des radicaux aliphatiques, alicycliques, ou arylaliphatiques contenant de 1 à 6 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 6 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique; A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH-.

De préférence, X⁻ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante: 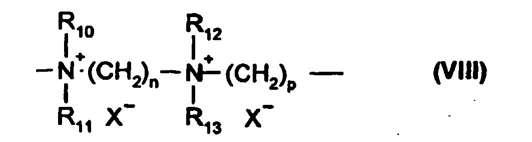 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(8) Les polymères de polyammonium quaternaire constitués de motifs de formule (IX) : 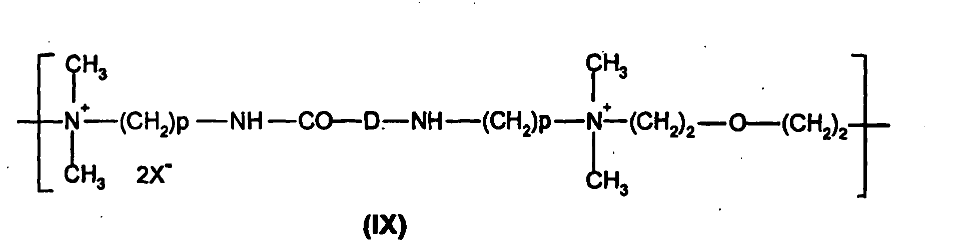 dans laquelle :
   p désigne un nombre entier variant de 1 à 6 environ,
   D peut être nul ou peut représenter un groupement -(CH₂)ᵣ -CO- dans lequel r désigne un nombre égal à 4 ou à 7, et
   X⁻ est un anion dérivé d'un acide minéral ou organique.

Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4 719 282.

Parmi ces polymères, on préfère ceux de masse moléculaire mesurée par RMN du Carbone 13 inférieure à 100000, et dans la formule de laquelle :
p est égal à 3, et,
   a) D représente un groupement -(CH₂)₄ -CO-, X désigne un atome de chlore, la masse moléculaire mesurée par RMN du Carbone 13 ( RMN¹³C ) étant d'environ 5600 ; un polymère de ce type est proposé par la société MIRANOL sous le nom de MIRAPOL-AD1,
   b) D représente un groupement -(CH₂)₇ -CO-, X désigne un atome de chlore, la masse moléculaire mesurée par RMN du Carbone 13 ( RMN¹³C ) étant d'environ 8100 ; un polymère de ce type est proposé par la société MIRANOL sous le nom de MIRAPOL-AZ1,
   c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
   d) un " Block Copolymer " formé de motifs correspondant aux polymères décrits aux alinéas a) et c), proposé par la société MIRANOL sous les noms MIRAPOL-9,
      (masse-moléculaire RMN¹³C, environ 7800) MIRAPOL-175, (masse moléculaire RMN¹³C, environ 8000) MIRAPOL-95, (masse moléculaire RMN¹³C, environ 12500).

Plus particulièrement encore, on préfère selon l'invention le polymère à motifs de formule (IX) dans laquelle p est égal à 3, D désigne la valeur zéro, X désigne un atome de chlore, la masse moléculaire mesurée par RMN du Carbone 13,
( RMN¹³C ) étant d'environ 25500.
(9) Les polymères quaternaires de vinylpyrrolidone et de vinylimidazole tels que par exemple les produits commercialisés sous les dénominations Luviquat FC 905, FC 550 et FC 370 par la société B.A.S.F.
(10) Les polyamines comme le Polyquart H vendu par HENKEL, référencé sous le nom de " POLYETHYLENEGLYCOL (15) TALLOW POLYAMINE " dans le dictionnaire CTFA.
(11) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de "SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 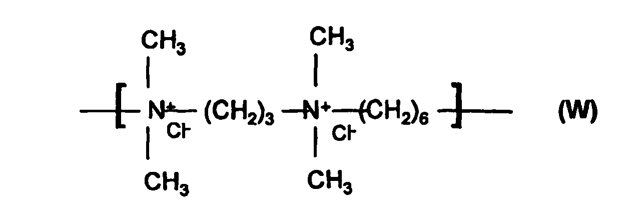 et notamment ceux dont le poids moléculaire, déterminé par chromatographie par perméation de gel, est compris entre 9500 et 9900; 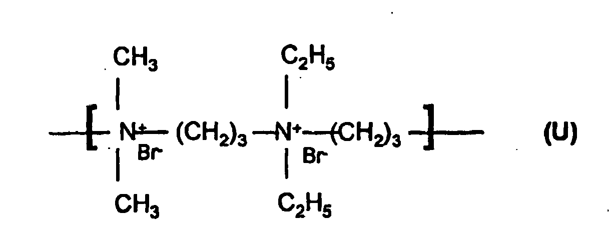 et notamment ceux dont le poids moléculaire, déterminé par chromatographie par perméation de gel, est d'environ 1200.

Le polymère cationique de densité de charge supérieure à 1 meq/g et ne comportant pas de chaîne grasse en C₈ - C₃₀ est présent dans la composition dans des proportions en poids de préférence comprises entre 0,1 et 10%, et encore de préférence entre 1 et 5% du poids total de la composition.

Les polymères associatifs sont des polymères comprenant au moins une chaîne grasse en C₈ - C₃₀ et dont les molécules sont capables, dans le milieu de formulation, de s'associer entre elles ou avec des molécules d'autres composés.

Les polymères associatifs selon l'invention sont choisis parmi les polymères associatifs, cationiques

Les polymères associatifs de type cationique utilisés dans la présente invention sont choisis parmi
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quaternisées à chaînes grasses en C₈-C₃₀, les hydroxyéthylcelluloses quaternisées modifiées par un groupement alkyle en C₁₂ ou C₁₈ tels que les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

Le ou les polymères associatifs sont présents dans la composition à des teneurs en poids comprises de préférence entre 0,05 et 10%, et encore plus préférentiellement entre 0,1 et 5% du poids total de la composition.

Le rapport en poids du composé cellulosique non ionique sans chaîne grasse en C₈ - C₃₀ sur le polymère associatif est de préférence compris entre 0,1 et 10, et plus préférentiellement entre 0,5 et 5.

Les colorants d'oxydation utilisables selon l'invention sont choisis parmi les bases d'oxydation et/ou les coupleurs.

De préférence les compositions selon l'invention contiennent au moins une base d'oxydation.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para-aminophénols, les bases hétérocycliques, ainsi que leurs sels d'addition avec un acide.

On peut notamment citer :
- (I) les paraphénylènediamines de formule (I) suivante et leurs sels d'addition avec un acide : 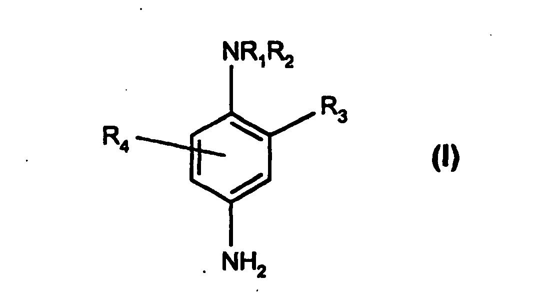 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

-(II) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 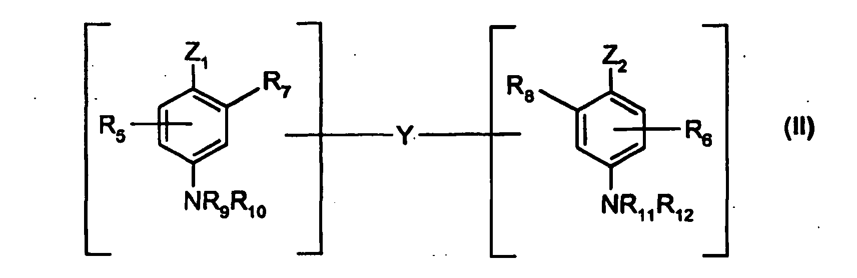 dans laquelle :
- Z₁ et Z₂, identiques ou différents, représentent un radical hydroxyle ou - NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

- (III) les para-aminophénols répondant à la formule (III) suivante, et leurs sels d'addition avec un acide : 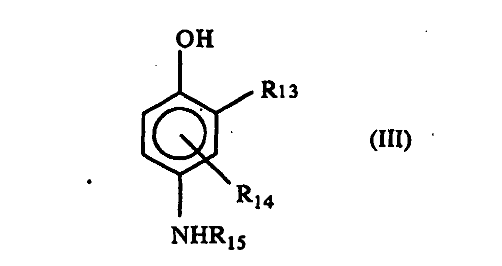 dans laquelle :
R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).
R₁₅ représente un atome d'hydrogène ou un radical alkyle en C₁-C₄.

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.

- (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

-(V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans le procédé de teinture selon l'invention sont ceux classiquement utilisés dans les compositions de teinture d'oxydation, c'est-à-dire les méta-aminophénols, les métaphénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-thiazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

La composition (A) et/ou la composition (B) peuvent en outre plus particulièrement contenir, au moins un polymère substantif amphotère différent des polymères associatifs de l'invention.

Les polymères substantifs amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un composé vinylique substitué contenant au moins un atome basique tel que plus particulièrement les dialkylaminoalkylméthacrylate et acrylate, les dialkylaminoalkyl-méthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique substitué contenant au moins un atome basique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 6 atomes de carbone et plus particulièrement le N-éthylacrylamide, le N-tertiobutyl-acrylamide, ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butyl-aminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   -[CO-R₁₉-CO-Z]- **(X)**

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 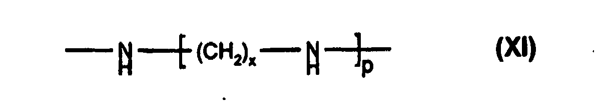 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 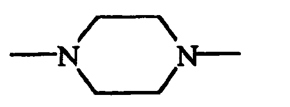
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 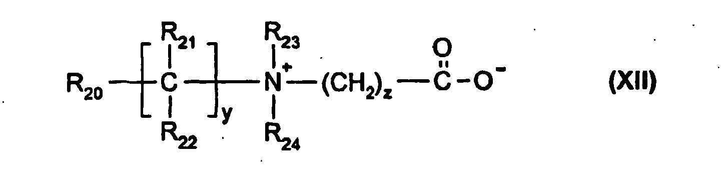 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent, un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 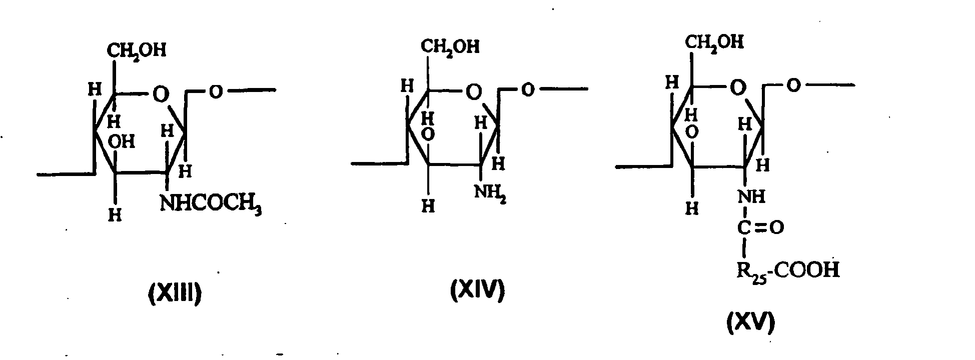 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 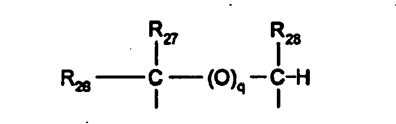 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.

Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical - CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 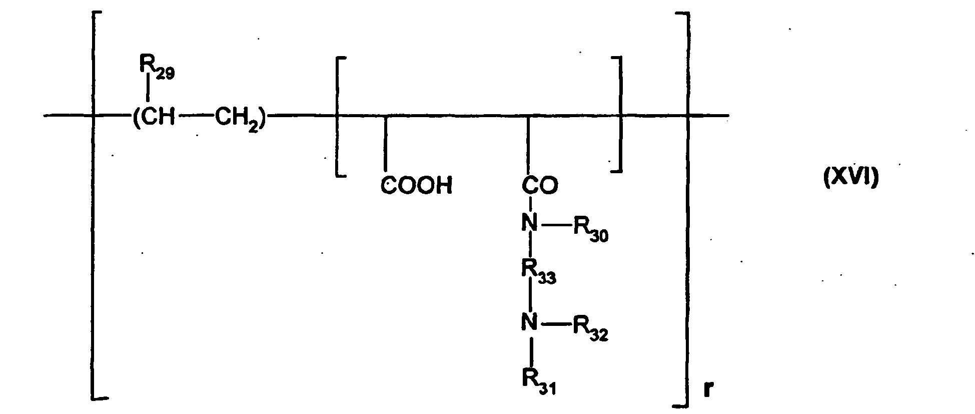 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : - R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone, r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- **(XVII)**

      où D désigne un radical 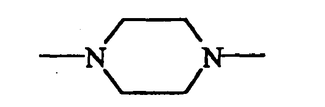 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- **(XVIII)**

      où D désigne un radical 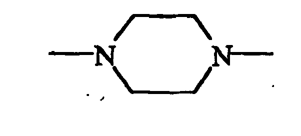 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères substantifs amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs.

Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :
(i) Tensioactif(s) anionique(s) :
   A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.
(ii) Tensioactif(s) non ionique(s) :
   Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.
(iii) Tensioactif(s) amphotère(s) ou zwittérionique(s) :
   Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle linéaire ou ramifié en C₅-C₂₀ provenant par exemple d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2, X' désigne le groupement -CH₂CH₂-COOH ou un atome d'hydrogène
   Y' désigne -COOH ou le radical -CH₂ - CHOH - SO₃H
   R₂' désigne un radical alkyle, linéaire ou ramifié, saturé ou non, en C₅-C₂₀, d'un acide R₉ -COOH présent par exemple dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, Sème édition, 1993, sous les dénominations Disodium Cocoamphodiacetate, Disodium Lauro-amphodiacetate, Disodium Caprylamphodiacetate, Disodium Caprylo-amphodiacetate, Disodium Cocoamphodipropionate, Disodium Lauro-amphodipropionate, Disodium Caprylamphodipropionate, Disodium Capryloamphodipropionate, Lauroamphodipropionic acid, Cocoampho-dipropionic acid.

A titre d'exemple on peut citer le cocoamphodiacetate commercialisé sous la dénomination commerciale MIRANOL^{®} C2M concentré par la société RHODIA CHIMIE.
(iv) Tensioactifs cationiques :
   Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Les compositions selon l'invention peuvent également contenir des agents d'ajustement de la rhéologie non associatifs tels que les épaississants cellulosiques
(hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.

Ces épaississants d'appoint peuvent représenter de 0,01 à 10% en poids du poids total de la composition.

Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol.

Les solvants peuvent alors être présents dans des concentrations comprises entre environ 0,5 et 20% et, de préférence, entre environ 2 et 10% en poids par rapport au poids total de la composition.

La composition (A) peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol.

Ladite composition peut également comprendre des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 1,5% en poids par rapport au poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi ou dans la composition (B), l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur on cofacteur respectif.

Le pH de la composition prête à l'emploi et appliquée sur les fibres kératiniques [composition résultant du mélange de la composition tinctoriale (A) et de la composition oxydante (B), est généralement compris entre les valeurs 4 et 11. Il est de préférence compris entre 6 et 10, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XIX) suivante : 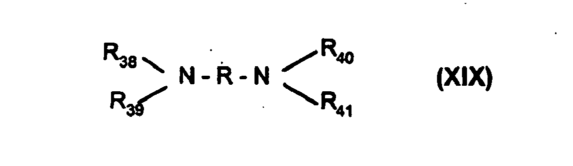 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions (A) et (B) décrites ci-avant, sur les fibres kératiniques sèches ou humides, et à la laisser agir pendant un temps de pause variant, de préférence, de 1 à 60 minutes, et plus préférentiellement de 10 à 45 minutes, à rincer les fibres, puis éventuellement à les laver au shampooing, puis à les rincer à nouveau, et à les sécher.

Une variante de ce procédé consiste à appliquer séquentiellement de manière décalée ou simultanée sur les fibres kératiniques sèches ou humides avec un éventuel rinçage intermédiaire une composition décrite ci-dessus et une composition comprenant un agent oxydant et à laisser agir lesdites compositions pendant un temps de pose variant de 1 à 60 minutes puis à rincer les fibres, puis éventuellement à les laver au shampooing puis à les rincer à nouveau et à les sécher.

## Revendications

1. Composition pour la teinture d'oxydation des fibres kératiniques, en particulier des fibres kératiniques humaines, telles que les cheveux, **caractérisée en ce qu'**elle comprend, dans un milieu approprié pour la teinture,
a) au moins un colorant d'oxydation,
b) au moins un polymère associatif cationique choisi parmi les celluloses quaternisées modifiées par des groupements comportant au moins une chaîne grasse et les hydroxyéthylcelluloses quaternisées modifiées par des groupements comportant au moins une chaîne grasse,
c) au moins un composé cellulosique non ionique ne comportant pas de chaîne grasse en C₈ - C₃₀, et
d) au moins un polymère cationique de densité de charge supérieure à 1 meq/g et ne comportant pas de chaîne grasse en C₈ - C₃₀.

2. Composition selon la revendication 1, **caractérisée en ce que** le composé cellulosique non ionique ne comportant pas de chaîne grasse en C₈ - C₃₀ est choisi parmi les celluloses non substituées et les éthers de cellulose.

3. Composition selon la revendication 2, **caractérisée en ce que** le composé cellulosique non ionique ne comportant pas de chaîne grasse en C₈ -C₃₀ est choisi parmi les alkyl(C₁-C₄)celluloses; les hydroxyalkyl(C₁-C₄)celluloses; les hydroxyéthylcelluloses et les hydroxypropylcelluloses ; les celluloses mixtes hydroxyalkyl(C₁-C₄)-alkyl (C₁-C₄)celluloses.

4. Composition selon l'une quelconque des revendications précédentes, **caractérisée en ce que** le composé cellulosique non ionique ne comportant pas de chaîne grasse en C₈-C₃₀ est présent dans la composition dans des proportions en poids comprises entre 0,1 et 10%, et de préférence entre 1 et 5% du poids total de la composition.

5. Composition selon l'une quelconque des revendications précédentes, **caractérisée en ce que** le polymère cationique de densité de charge supérieure à 1 meq/g et ne comportant pas de chaîne grasse en C₈ - C₃₀ est l'homopolymère de chlorure de diméthyldiallylammonium.

6. Composition selon l'une quelconque des revendications 1 à 4, **caractérisée en ce que** le polymère cationique de densité de charge supérieure à 1 meq/g et ne comportant pas de chaîne grasse en C₈-C₃₀ est un polymère aux motifs récurrents répondant à la formule (W) suivante :

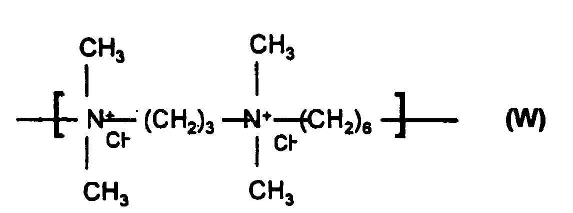

7. Composition selon l'une quelconque des revendications 1 à 4, **caractérisée en ce que** le polymère cationique de densité de charge supérieure à 1 meq/g et ne comportant pas de chaîne grasse en C₈- C₃₀ est un polymère aux motifs récurrents répondant à la formule (U) suivante :

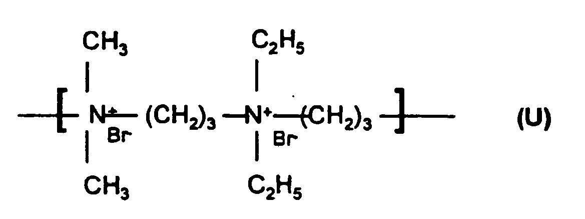

8. Composition selon l'une quelconque des revendications précédentes, **caractérisée en ce que** le polymère cationique de densité de charge supérieure à 1 meq/g et ne comportant pas de chaîne grasse en C₈ - C₃₀ est présent dans la composition dans des proportions en poids comprises entre 0,1 et 10%, et de préférence entre 1 et 5% du poids total de la composition.

9. Composition selon la revendication 1, **caractérisée en ce que** les groupes alkyle des celluloses ou des hydroxyéthylcelluloses quaternisées comportent de 8 à 30 atomes de carbone.

10. Composition selon les revendications précédentes, **caractérisée en ce que** le polymère amphiphile cationique est une hydroxyéthylcellulose quaternisée modifiée par un groupement alkyle en C₁₂ ou C₁₈.

11. Composition selon l'une quelconque des revendications précédentes, **caractérisée en ce que** le ou les polymères associatifs sont présents dans la composition à des teneurs en poids comprises entre 0,05 et 10%, et encore de préférence entre 0,1 et 5% du poids total de la composition.

12. Composition selon l'une quelconque des revendications précédentes, **caractérisée en ce que** le rapport en poids du composé cellulosique non ionique ne comportant pas de chaîne grasse en C₈-C₃₀ sur le polymère associatif est compris entre 0,1 et 10, plus préférentiellement entre 0,5 et 5.

13. Composition selon l'une quelconque des revendications précédentes, **caractérisée en ce que** le colorant d'oxydation est choisi parmi les bases d'oxydation et/ou les coupleurs.

14. Composition selon la revendication 13, **caractérisée en ce qu'**elle comprend au moins une base d'oxydation.

15. Composition selon la revendication 13 ou 14, **caractérisée en ce que** les bases d'oxydation sont choisies parmi les ortho- et para-phénylènediamines, les bases doubles, les ortho- et para-aminophénols, les bases hétérocycliques, ainsi que les sels d'addition de ces composés avec un acide.

16. Composition selon la revendication 15, **caractérisée en ce que** les para-phénylènediamines sont choisies parmi les composés de structure (I) suivante : 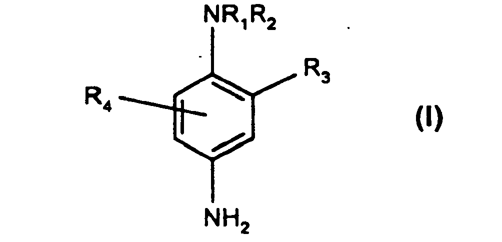 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C4)alkyle(C₁-C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido :
- R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoaolcoxy en C₁-C₄ ou carbamoylaminoalcoxy en C₁-C₄,
- R₄ représente un atome d'hydrogène, d'halogène ou un radical alkyle en C₁-C₄.

17. Composition selon la revendication 15, **caractérisée en ce que** les bases doubles sont choisies parmi les composés de structure (II) suivante : 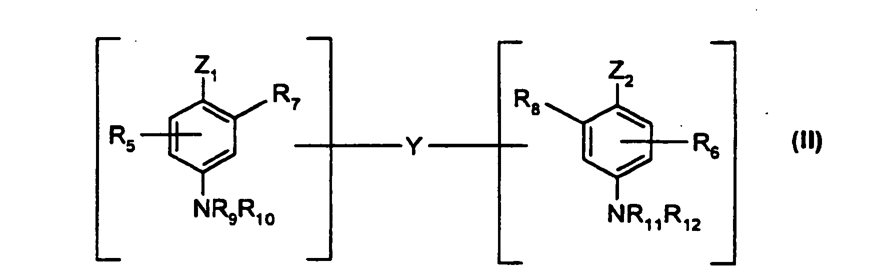 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un plusieurs groupements azotés et/ou par un ou plusieurs hétéroatomes, et éventuellement substituée par un plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄;
étant entendu que les composés de formule (II) ne comportent qu'un seul bras de liaison Y par molécule.

18. Composition selon la revendication 15, **caractérisée en ce que** les para-aminophénols sont choisis parmi les composés de structure (III) suivante : 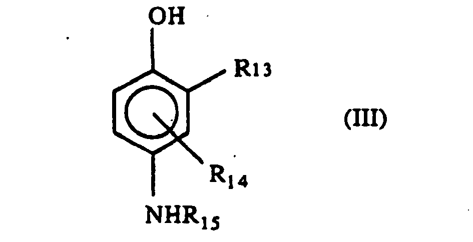 dans laquelle :
R₁₃ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl (C₁-C₄)aminoalkyle en C₁-C₄,
R₁₄ représente un atome d'hydrogène, un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
R₁₅ représente un atome d'hydrogène ou un radical alkyle en C₁-C₄.

19. Composition selon la revendication 15, **caractérisée en ce que** les bases hétérocycliques sont choisies parmi les dérivés pyridiniques, les dérivés pyrimidiniques, les dérivés pyrazoliques.

20. Composition selon l'une quelconque des revendications 13 a 19. **caractérisée en ce que** les bases d'oxydation représentent de 0,0005 à 12%, et de préférence de 0,005 à 8% en poids du poids total de la composition.

21. Composition selon la revendication 13, **caractérisée en ce que** les coupleurs sont choisis parmi les métaphénylènediamines, les méta-aminophénols, les métadiphénols, les coupleurs hérérocycliques, et les sels d'addition de ces composés avec un acide.

22. Composition selon la revendication 13 ou 21, **caractérisée en ce que** les coupleurs représentent de 0,0001 à 10%, et de préférence de 0,005 à 5% en poids du poids total de la composition.

23. Composition selon l'une quelconque des revendications 13 à 22, **caractérisée en ce** les sels d'addition avec un acide des colorants d'oxydation sont choisis parmi les chlorhydrates, les bromhydrates, les sulfates, les tartrates, les lactates et les acétates.

24. Composition selon l'une quelconque des revendications précédentes, **caractérisée en ce qu'**elle comprend en outre des colorants directs.

25. Composition selon l'une quelconque des revendications précidentes, **caractérisée en ce qu'**elle comprend au moins un tensioactif choisi parmi les tensioactifs anioniques, amphotères, non ioniques, zwitterioniques et cationiques.

26. Composition selon la revendication 25, **caractérisée en ce que** le tensioactif est non ionique.

27. Composition selon la revendication 25 ou 26, **caractérisée en ce que** les tensioactifs représentent de 0,01 à 40% et de préférence de 0,5 à 30% en poids, du poids total de la composition.

28. composition selon l'une quelconque des revendications précédentes, **caractérisée en ce qu'**elle comprend au moins un épaississant d'appoint.

29. Composition selon la revendication 28, **caractérisée en ce que** l'épaississant d'appoint est un épaississant cellulosique, un dérivé de gomme de guar, une gomme d'origine microbienne, un épaississant synthétique.

30. Composition selon la revendication 28 ou 29, **caractérisée en ce que** le ou les épaississants d'appoint représentent de 0,01 à 10% en poids du poids total de la composition.

31. Composition selon l'une quelconque des revendications précédentes, **caractérisée en ce qu'**elle comprend en outre au moins un agent réducteur, dans des quantités allant de 0,05 à 1,5% en poids par rapport au poids total de la composition.

32. Composition prête à l'emploi selon l'une quelconque des revendications précédentes, **caractérisée en ce qu'**elle comprend en outre un agent oxydant.

33. Composition selon la revendication 32, **caractérisée en ce que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

34. Composition selon la revendication 32, **caractérisée en ce que** l'agent oxydant est le peroxyde d'hydrogène.

35. Composition selon la revendication 34, **caractérisée en ce que** l'agent oxydant est une solution d'eau oxygénée dont le titre varie de 1 à 40 volumes.

36. composition selon la revendication 35, **caractérisée en ce qu'**elle possède un pH allant de 4 à 11.

## Claims

1. Composition for the oxidation dyeing of keratin fibres, in particular of human keratin fibres such as the hair, **characterized in that** it comprises, in a medium that is suitable for dyeing:
a) at least one oxidation dye,
b) at least one cationic associative polymer chosen from quaternized celluloses modified with groups comprising at least one fatty chain and quaternized hydroxyethylcelluloses modified with groups comprising at least one fatty chain,
c) at least one nonionic cellulose-based compound not comprising a C₈-C₃₀ fatty chain, and
d) at least one cationic polymer with a charge density of greater than 1 meq/g and not comprising a C₈-C₃₀ fatty chain.

2. Composition according to Claim 1, **characterized in that** the nonionic cellulose-based compound not comprising a C₈-C₃₀ fatty chain is chosen from unsubstituted celluloses and cellulose ethers.

3. Composition according to Claim 2, **characterized in that** the nonionic cellulose-based compound not comprising a C₈-C₃₀ fatty chain is chosen from (C₁-C₄)alkylcelluloses; hydroxy (C₁-C₄) alkylcelluloses; hydroxyethylcelluloses and hydroxypropylcelluloses; and mixed hydroxy (C₁-C₄) alkyl (C₁-C₄) alkylcelluloses.

4. Composition according to any one of the preceding claims, **characterized in that** the nonionic cellulose-based compound not comprising a C₈-C₃₀ fatty chain is present in the composition in weight proportions of between 0.1% and 10% and preferably between 1% and 5% relative to the total weight of the composition.

5. Composition according to any one of the preceding claims, **characterized in that** the cationic polymer with a charge density of greater than 1 meq/g and not comprising a C₈-C₃₀ fatty chain is dimethyldiallyl-ammonium chloride homopolymer.

6. Composition according to any one of Claims 1 to 4, **characterized in that** the cationic polymer with a charge density of greater than 1 meq/g and not comprising a C₈-C₃₀ fatty chain is a polymer containing repeating units corresponding to formula (W) below:

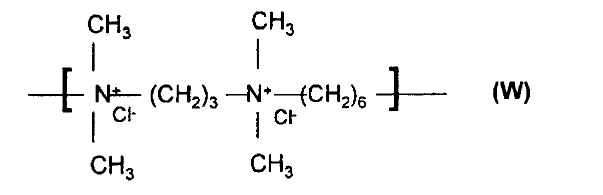

7. Composition according to any one of Claims 1 to 4, **characterized in that** the cationic polymer with a charge density of greater than 1 meq/g and not comprising a C₈-C₃₀ fatty chain is a polymer containing repeating units corresponding to formula (U) below:

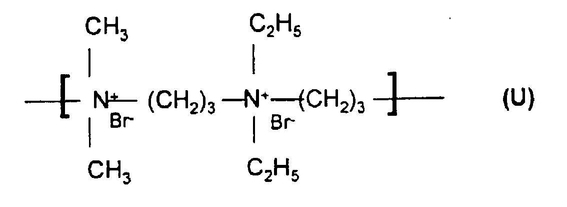

8. Composition according to any one of the preceding claims, **characterized in that** the cationic polymer with a charge density of greater than 1 meq/g and not comprising a C₈-C₃₀ fatty chain is present in the composition in weight proportions of between 0.1% and 10% and preferably between 1% and 5% relative to the total weight of the composition.

9. Composition according to Claim 1, **characterized in that** the alkyl groups of the quaternized celluloses or hydroxyethylcelluloses contain from 8 to 30 carbon atoms.

10. Composition according to the preceding claims, **characterized in that** the cationic amphiphilic polymer is a quaternized hydroxyethylcellulose modified with a C₁₂ or C₁₈ alkyl group.

11. Composition according to any one of the preceding claims, **characterized in that** the associative polymer(s) is (are) present in the composition in weight contents of between 0.05% and 10% and more preferably between 0.1% and 5% of the total weight of the composition.

12. Composition according to any one of the preceding claims, **characterized in that** the weight ratio of the nonionic cellulose-based compound not comprising a C₈-C₃₀ fatty chain to the associative polymer is between 0.1 and 10 and more preferably between 0.5 and 5.

13. Composition according to any one of the preceding claims, **characterized in that** the oxidation dye is chosen from the oxidation bases and/or couplers.

14. Composition according to Claim 13, **characterized in that** it comprises at least one oxidation base.

15. Composition according to Claim 13 or 14, **characterized in that** the oxidation bases are chosen from ortho- and para-phenylenediamines, double bases, ortho- and para-aminophenols and heterocyclic bases, and also the addition salts of these compounds with an acid.

16. Composition according to Claim 15, **characterized in that** the para-phenylenediamines are chosen from the compounds of structure (I) below: 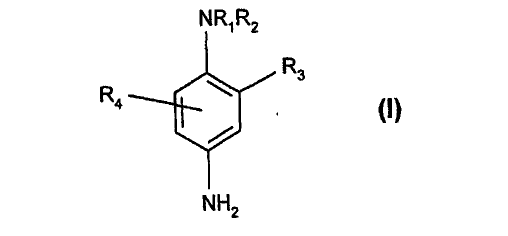 in which:
- R₁ represents a hydrogen atom, a C₁-C₄ alkyl radical, a C₁-C₄ monohydroxyalkyl radical, a C₂-C₄ polyhydroxyalkyl radical, a (C₁-C₄)alkoxy (C₁-C₄) alkyl radical or a C₁-C₄ alkyl radical substituted with a nitrogenous, phenyl or 4'-aminophenyl group;
- R₂ represents a hydrogen atom, a C₁-C₄ alkyl radical, a C₁-C₄ monohydroxyalkyl radical, a C₂-C₄ polyhydroxyalkyl radical, a (C₁-C₄) alkoxy (C₁-C₄) alkyl radical or a C₁-C₄ alkyl radical substituted with a nitrogenous group;
R₁ and R₂ may also form, with the nitrogen atom that bears them, a 5- or 6-membered nitrogenous heterocycle optionally substituted with one or more alkyl, hydroxyl or ureido groups;
- R₃ represents a hydrogen atom, a halogen atom such as a chlorine atom, a C₁-C₄ alkyl radical, a sulfo radical, a carboxy radical, a C₁-C₄ monohydroxyalkyl radical, a C₁-C₄ hydroxyalkoxy radical, an acetylamino (C₁-C₄) alkoxy radical, a mesylamino(C₁-C₄)alkoxy radical or a carbamoylamino (C₁-C₄) alkoxy radical,
- R₄ represents a hydrogen or halogen atom or a C₁-C₄ alkyl radical.

17. Composition according to Claim 15, **characterized in that** the double bases are chosen from the compounds of structure (II) below: 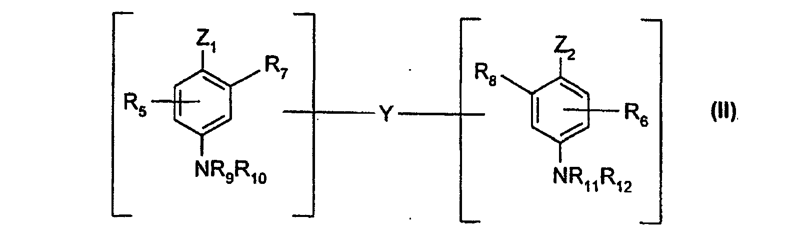 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more hetero atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (II) contain only one linker arm Y per molecule.

18. Composition according to Claim 15, **characterized in that** the para-aminophenols are chosen from the compounds of formula (III) below: 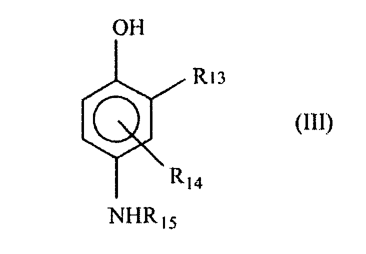 in which:
R₁₃ represents a hydrogen atom, a halogen atom, such as fluorine, or a C₁-C₄ alkyl, C₁-C₄ monohydroxyalkyl, (C₁-C₄) alkoxy (C₁-C₄) alkyl, C₁-C₄ aminoalkyl or hydroxy (C₁-C₄) alkylamino (C₁-C₄) alkyl radical,
R₁₄ represents a hydrogen atom, a halogen atom, such as fluorine, or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄) alkoxy (C₁-C₄) alkyl radical,
R₁₅ represents a hydrogen atom or a C₁-C₄ alkyl radical.

19. Composition according to Claim 15, **characterized in that** the heterocyclic bases are chosen from pyridine derivatives, pyrimidine derivatives and pyrazole derivatives.

20. Composition according to any one of Claims 13 to 19, **characterized in that** the oxidation bases represent from 0.0005% to 12% and preferably from 0.005% to 8% by weight relative to the total weight of the composition.

21. Composition according to Claim 13, **characterized in that** the couplers are chosen from metaphenylenediamines, meta-aminophenols, meta-diphenols and heterocyclic couplers, and the addition salts of these compounds with an acid.

22. Composition according to Claim 13 or 21, **characterized in that** the couplers represent from 0.0001% to 10% and preferably from 0.005% to 5% by weight relative to the total weight of the composition.

23. Composition according to any one of Claims 13 to 22, **characterized in that** the addition salts with an acid of the oxidation dyes are chosen from the hydrochlorides, hydrobromides, sulfates, tartrates, lactates and acetates.

24. Composition according to any one of the preceding claims, **characterized in that** it also comprises direct dyes.

25. Composition according to any one of the preceding claims, **characterized in that** it comprises at least one surfactant chosen from anionic, amphoteric, nonionic, zwitterionic and cationic surfactants.

26. Composition according to Claim 25, **characterized in that** the surfactant is nonionic.

27. Composition according to Claim 25 or 26, **characterized in that** the surfactants represent from 0.01% to 40% and preferably from 0.5% to 30% by weight relative to the total weight of the composition.

28. Composition according to any one of the preceding claims, **characterized in that** it comprises at least one additional thickener.

29. Composition according to Claim 28, **characterized in that** the additional thickener is a cellulose-based thickener, a guar gum derivative, a gum of microbial origin or a synthetic thickener.

30. Composition according to Claim 28 or 29, **characterized in that** the additional thickener(s) represent(s) from 0.01% to 10% by weight relative to the total weight of the composition.

31. Composition according to any one of the preceding claims, **characterized in that** it also comprises at least one reducing agent, in amounts ranging from 0.05% to 1.5% by weight relative to the total weight of the composition.

32. Ready-to-use composition according to any one of the preceding claims, **characterized in that** it also comprises an oxidizing agent.

33. Composition according to Claim 32, **characterized in that** the oxidizing agent is chosen from hydrogen peroxide, urea peroxide, alkali metal bromates or ferricyanides, persalts, and redox enzymes, optionally with the respective donor or cofactor thereof.

34. Composition according to Claim 32, **characterized in that** the oxidizing agent is hydrogen peroxide.

35. Composition according to Claim 34, **characterized in that** the oxidizing agent is an aqueous hydrogen peroxide solution whose titre ranges from 1 to 40 volumes.

36. Composition according to Claim 35, **characterized in that** it has a pH ranging from 4 to 11.

## Patentansprüche

1. Zusammensetzung zur oxidativen Färbung von Keratinfasern, insbesondere von menschlichen Keratinfasern, wie etwa Haaren, **dadurch gekennzeichnet, dass** sie in einem für die Färbung geeigneten Medium Folgendes umfasst:
a) mindestens einen Oxidationsfarbstoff,
b) mindestens ein kationisches assoziatives Polymer, welches aus quaternisierten Cellulosen, die mit Gruppen modifiziert sind, die mindestens eine Fettkette aufweisen, und quaternisierten Hydroxyethylcellulosen, die mit Gruppen modifiziert sind, die mindestens eine Fettkette aufweisen, ausgewählt ist,
c) mindestens eine nichtionische Celluloseverbindung, die keine C₈-C₃₀-Fettkette aufweist, und
d) mindestens ein kationisches Polymer mit einer Ladungsdichte, die größer als 1 mÄq/g ist, welches keine C₈-C₃₀-Fettkette aufweist.

2. Zusammensetzung nach Anspruch 1, **dadurch gekennzeichnet, dass** die nichtionische Celluloseverbindung, die keine C₈-C₃₀-Fettkette aufweist, aus den nichtsubstituierten Cellulosen und den Celluloseethern ausgewählt ist.

3. Zusammensetzung nach Anspruch 2, **dadurch gekennzeichnet, dass** die nichtionische Celluloseverbindung, die keine C₈-C₃₀-Fettkette aufweist, aus den (C₁-C₄)-Alkylcellulosen; (C₁-C₄)-Hydroxyalkylcellulosen; Hydroxyethylcellulosen und Hydroxypropylcellulosen; den gemischten Cellulosen (C₁-C₄) -Hydroxyalkyl- (C₁-C₄) -alkylcellulosen ausgewählt ist.

4. Zusammensetzung nach einem der vorhergehenden Ansprüche, **dadurch gekennzeichnet, dass** die nichtionische Celluloseverbindung, die keine C₈-C₃₀-Fettkette aufweist, in der Zusammensetzung in Gewichtsanteilen vorhanden ist, die zwischen 0,1 und 10 % und vorzugsweise zwischen 1 und 5 % des Gesamtgewichts der Zusammensetzung liegen.

5. Zusammensetzung nach einem der vorhergehenden Ansprüche, **dadurch gekennzeichnet, dass** das kationische Polymer mit einer Ladungsdichte, die größer als 1 mÄq/g ist, welches keine C₈-C₃₀-Fettkette aufweist, das Homopolymer von Dimethyldiallylammoniumchlorid ist.

6. Zusammensetzung nach einem der Ansprüche 1 bis 4, **dadurch gekennzeichnet, dass** das kationische Polymer mit einer Ladungsdichte, die größer als 1 mÄq/g ist, welches keine C₈-C₃₀-Fettkette aufweist, ein Polymer mit wiederkehrenden Einheiten ist, welche der folgenden Formel (W) genügen:

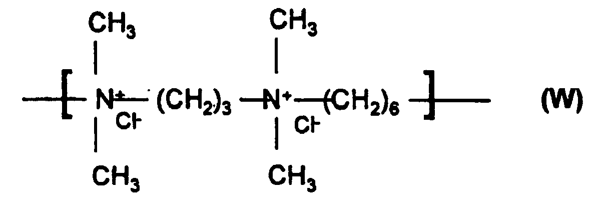

7. Zusammensetzung nach einem der Ansprüche 1 bis 4, **dadurch gekennzeichnet, dass** das kationische Polymer mit einer Ladungsdichte, die größer als 1 mÄq/g ist, welches keine C₈-C₃₀-Fettkette aufweist, ein Polymer mit wiederkehrenden Einheiten ist, welche der folgenden Formel (U) genügen:

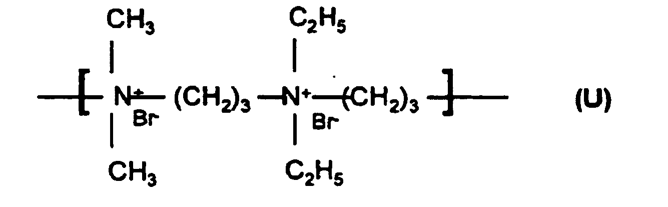

8. Zusammensetzung nach einem der vorhergehenden Ansprüche, **dadurch gekennzeichnet, dass** das kationische Polymer mit einer Ladungsdichte, die größer als 1 mÄq/g ist, welches keine C₈-C₃₀-Fettkette aufweist, in der Zusammensetzung in Gewichtsanteilen vorhanden ist, die zwischen 0,1 und 10 % und vorzugsweise zwischen 1 und 5 % des Gesamtgewichts der Zusammensetzung liegen.

9. Zusammensetzung nach Anspruch 1, **dadurch gekennzeichnet, dass** die Alkylgruppen der quaternisierten Cellulosen oder Hydroxyethylcellulosen 8 bis 30 Kohlenstoffatome aufweisen.

10. Zusammensetzung nach den vorhergehenden Ansprüchen, **dadurch gekennzeichnet, dass** das kationische amphiphile Polymer eine quaternisierte Hydroxyethylcellulose ist, die durch eine C₁₂- oder C₁₈-Alkylgruppe modifiziert ist.

11. Zusammensetzung nach einem der vorhergehenden Ansprüche, **dadurch gekennzeichnet, dass** das oder die assoziative(n) Polymer(e) in der Zusammensetzung in Gewichtsanteilen vorhanden ist (sind), die zwischen 0,05 und 10 % und vorzugsweise zwischen 0,1 und 5 % des Gesamtgewichts der Zusammensetzung liegen.

12. Zusammensetzung nach einem der vorhergehenden Ansprüche, **dadurch gekennzeichnet, dass** das Gewichtsverhältnis der nichtionischen Celluloseverbindung, die keine C₈-C₃₀-Fettkette aufweist, zu dem assoziativen Polymer zwischen 0,1 und 10, stärker bevorzugt zwischen 0,5 und 5 liegt.

13. Zusammensetzung nach einem der vorhergehenden Ansprüche, **dadurch gekennzeichnet, dass** der Oxidationsfarbstoff aus den Oxidationsbasen und/oder den Kopplern ausgewählt ist.

14. Zusammensetzung nach Anspruch 13, **dadurch gekennzeichnet, dass** sie mindestens eine Oxidationsbase umfasst.

15. Zusammensetzung nach Anspruch 13 oder 14, **dadurch gekennzeichnet, dass** die Oxidationsbasen aus den ortho- und para-Phenylendiaminen, Doppelbasen, ortho- und para-Aminophenolen, heterozyklischen Basen sowie den Säureadditionssalzen dieser Verbindungen ausgewählt sind.

16. Zusammensetzung nach Anspruch 15, **dadurch gekennzeichnet, dass** die para-Phenylendiamine aus den Verbindungen mit der folgenden Struktur (I) ausgewählt sind: 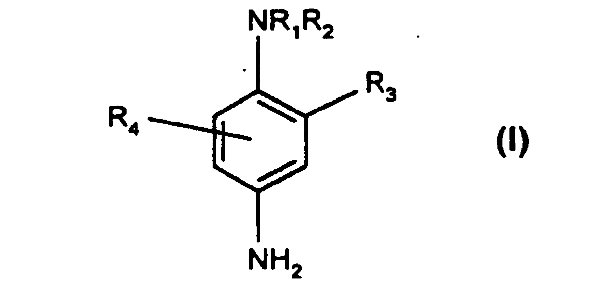 worin:
- R₁ ein Wasserstoffatom, ein C₁-C₄-Alkylradikal, C₁-C₄-Monohydroxyalkyl, C₂-C₄-Polyhydroxyalkyl, (C₁-C₄) -Alkoxy- (C₁-C₄) -alkyl, ein mit einer stickstoffhaltigen Gruppe substituiertes C₁-C₄-Alkyl, Phenyl oder 4'-Aminophenyl darstellt;
- R₂ ein Wasserstoffatom, ein C₁-C₄-Alkylradikal, C₁-C₄-Monohydroxyalkyl oder C₂-C₄-Polyhydroxyalkyl, (C₁-C₄) -Alkoxy- (C₁-C₄) - alkyl oder ein mit einer stickstoffhaltigen Gruppe substituiertes C₁-C₄-Alkyl darstellt;
R₁ und R₂ auch mit dem Stickstoffatom, welches sie trägt, einen stickstoffhaltigen Heterozyklus mit 5 oder 6 Kettengliedern bilden können, der eventuell mit einer oder mehreren Alkyl-, Hydroxy- oder Ureidogruppen substituiert ist;
- R₃ ein Wasserstoffatom, ein Halogenatom wie etwa ein Chloratom, ein C₁-C₄-Alkylradikal, Sulfo, Carboxy, C₁-C₄-Monohydroxyalkyl oder C₁-C₄-Hydroxyalkoxy, C₁-C₄-Acetylaminoalkoxy, C₁-C₄-Mesylaminoalkoxy oder C₁-C₄-Carbamoylaminoalkoxy darstellt,
- R₄ ein Wasserstoffatom, ein Halogenatom oder ein C₁-C₄-Alkylradikal darstellt.

17. Zusammensetzung nach Anspruch 15, **dadurch gekennzeichnet, dass** die Doppelbasen aus den Verbindungen mit der folgenden Struktur (II) ausgewählt sind: 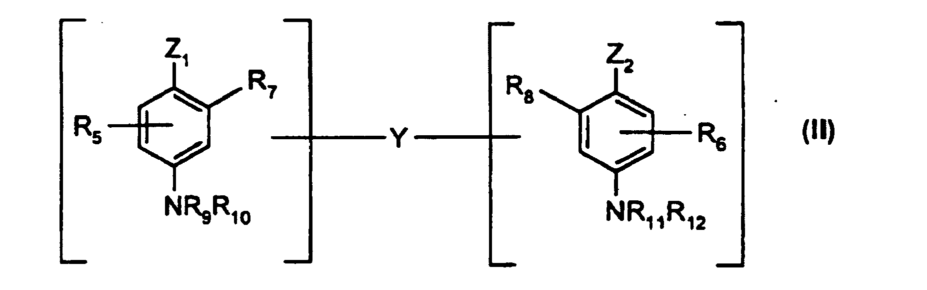 worin:
- Z₁ und Z₂, die identisch oder verschieden sind, ein Hydroxylradikal oder -NH₂ darstellen, das mit einem C₁-C₄-Alkylradikal oder mit einer Brückengruppe Y substituiert sein kann;
- die Brückengruppe Y eine geradkettige oder verzweigte Alkylenkette mit 1 bis 14 Kohlenstoffatomen darstellt, die durch eine oder mehrere stickstoffhaltige Gruppen und/oder durch ein oder mehrere Heteroatome unterbrochen oder beendet sein kann und eventuell mit einem oder mehreren C₁-C₆-Hydroxyl- oder Alkoxyradikalen substituiert ist;
- R₅ und R₆ ein Wasserstoff- oder Halogenatom, ein C₁-C₄-Alkylradikal, C₁-C₄-Monohydroxyalkyl, C₂-C₄-Polyhydroxyalkyl, C₁-C₄-Aminoalkyl oder eine Brückengruppe Y darstellen;
- R₇, R₈, R₉, R₁₀, R₁₁ und R₁₂, die identisch oder verschieden sind, ein Wasserstoffatom, eine Brückengruppe Y oder ein C₁-C₄-Alkylradikal darstellen;
mit der Maßgabe, dass die Verbindungen mit der Formel (II) nur eine einzige Brückengruppe Y pro Molekül aufweisen.

18. Zusammensetzung nach Anspruch 15, **dadurch gekennzeichnet, dass** die para-Aminophenole aus den Verbindungen mit der folgenden Struktur (III) ausgewählt sind: 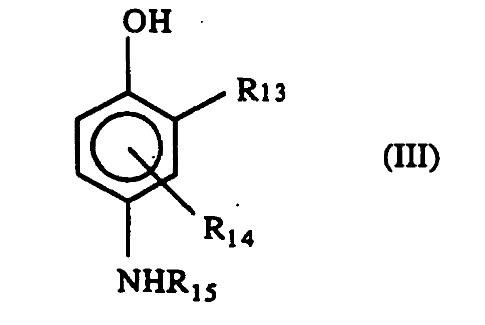 worin:
R₁₃ ein Wasserstoffatom, ein Halogenatom wie etwa Fluor, ein C₁-C₄-Alkylradikal, C₁-C₄-Monohydroxyalkyl, (C₁-C₄)-Alkoxy-(C₁-C₄)-alkyl oder C₁-C₄-Aminoalkyl oder (C₁-C₄)-Hydroxyalkyl-(C₁-C₄)-aminoalkyl darstellt,
R₁₄ ein Wasserstoffatom, ein Halogenatom wie etwa Fluor, ein C₁-C₄-Alkylradikal, C₁-C₄-Monohydroxyalkyl, C₂-C₄-Polyhydroxyalkyl, C₁-C₄-Aminoalkyl, C₁-C₄-Cyanoalkyl oder (C₁-C₄)-Alkoxy-(C₁-C₄)-alkyl darstellt,
R₁₅ ein Wasserstoffatom oder ein C₁-C₄-Alkylradikal darstellt.

19. Zusammensetzung nach Anspruch 15, **dadurch gekennzeichnet, dass** die heterozyklischen Basen aus den Pyridinderivaten, Pyrimidinderivaten und Pyrazolderivaten ausgewählt sind.

20. Zusammensetzung nach einem der Ansprüche 13 bis 19, **dadurch gekennzeichnet, dass** die Oxidationsbasen 0,0005 bis 12 Gew.-% und vorzugsweise 0,005 bis 8 Gew.-% des Gesamtgewichts der Zusammensetzung ausmachen.

21. Zusammensetzung nach Anspruch 13, **dadurch gekennzeichnet, dass** die Koppler aus Metaphenylendiaminen, meta-Aminophenolen, Metadiphenolen, heterozyklischen Kopplern und Säureadditionssalzen dieser Verbindungen ausgewählt sind.

22. Zusammensetzung nach Anspruch 13 oder 21, **dadurch gekennzeichnet, dass** die Koppler 0,0001 bis 10 Gew.-% und vorzugsweise 0,005 bis 5 Gew.-% des Gesamtgewichts der Zusammensetzung ausmachen.

23. Zusammensetzung nach einem der Ansprüche 13 bis 22, **dadurch gekennzeichnet, dass** die Säureadditionssalze der Oxidationsfarbstoffe aus den Hydrochloriden, Hydrobromiden, Sulfaten, Tartraten, Lactaten und Acetaten ausgewählt sind.

24. Zusammensetzung nach einem der vorhergehenden Ansprüche, **dadurch gekennzeichnet, dass** sie außerdem Direktfarbstoffe umfasst.

25. Zusammensetzung nach einem der vorhergehenden Ansprüche, **dadurch gekennzeichnet, dass** sie mindestens ein Tensid umfasst, das aus den anionischen, amphoteren, nichtionischen, zwitterionischen und kationischen Tensiden ausgewählt ist.

26. Zusammensetzung nach Anspruch 25, **dadurch gekennzeichnet, dass** das Tensid nichtionisch ist.

27. Zusammensetzung nach Anspruch 25 oder 26, **dadurch gekennzeichnet, dass** die Tenside 0,01 bis 40 Gew.-% und vorzugsweise 0,5 bis 30 Gew.-% des Gesamtgewichts der Zusammensetzung ausmachen.

28. Zusammensetzung nach einem der vorhergehenden Ansprüche, **dadurch gekennzeichnet, dass** sie mindestens einen Verdickungsmittelzusatz umfasst.

29. Zusammensetzung nach Anspruch 28, **dadurch gekennzeichnet, dass** der Verdickungsmittelzusatz ein cellulosisches Verdickungsmittel, ein Guargummiderivat, ein Gummi mikrobiellen Ursprungs oder ein synthetisches Verdickungsmittel ist.

30. Zusammensetzung nach Anspruch 28 oder 29, **dadurch gekennzeichnet, dass** der Verdickungsmittelzusatz oder die Verdickungsmittelzusätze 0,01 bis 10 Gew.-% des Gesamtgewichts der Zusammensetzung ausmachen.

31. Zusammensetzung nach einem der vorhergehenden Ansprüche, **dadurch gekennzeichnet, dass** sie außerdem mindestens ein Reduktionsmittel umfasst, in Mengen von 0,05 bis 1,5 Gew.-%, bezogen auf das Gesamtgewicht der Zusammensetzung.

32. Gebrauchsfertige Zusammensetzung nach einem der vorhergehenden Ansprüche, **dadurch gekennzeichnet, dass** sie außerdem ein Oxidationsmittel umfasst.

33. Zusammensetzung nach Anspruch 32, **dadurch gekennzeichnet, dass** das Oxidationsmittel aus Wasserstoffperoxid, Harnstoffperoxid, Bromaten oder Ferricyaniden von Alkalimetallen, Salzen von Persäuren und Oxidoreduktasen, eventuell mit ihrem jeweiligen Donor oder Cofaktor, ausgewählt ist.

34. Zusammensetzung nach Anspruch 32, **dadurch gekennzeichnet, dass** das Oxidationsmittel Wasserstoffperoxid ist.

35. Zusammensetzung nach Anspruch 34, **dadurch gekennzeichnet, dass** das Oxidationsmittel eine Wasserstoffperoxidlösung mit einem Titer von 1 bis 40 Volumina ist.

36. Zusammensetzung nach Anspruch 35, **dadurch gekennzeichnet, dass** sie einen pH-Wert von 4 bis 11 aufweist.
